# EUROPEAN PATENT SPECIFICATION

(11) **EP 1 819 354 B1**
(45) Date of publication and mention of the grant of the patent: **20.07.2011**
(21) Application number: 05791454.1
(22) Date of filing: 26.08.2005
(51) Int. Cl.: A61K 38/17, C07K 14/72, G01N 33/50

(54) **METHODS AND COMPOUNDS FOR TREATING DIABETES**
VERFAHREN UND VERBINDUNGEN ZUR BEHANDLUNG VON DIABETES
METHODES ET COMPOSES POUR TRAITER LE DIABETE

(30) Priority: 26.08.2004 US 604792 P
(43) Date of publication of application: 22.08.2007
(73) Proprietor: BioCrine AB, 112 22 Stockholm (SE)
(72) Inventor: BERGGREN, Per, Olof, S-16955 Solna (SE); BERGGREN, Lisa-Juntti, SE-16955 Solna (SE)
(74) Representative: Zwicker, Jörk
(86) International application number: PCT/EP2005/009238
(87) International publication number: WO 2006/021451

(56) References cited:
- WO-A-2004/056315
- ANDO Y ET AL: "Down regulation of a harmful variant protein by replacement of its normal protein" BIOCHIMICA ET BIOPHYSICA ACTA. MOLECULAR BASIS OF DISEASE, AMSTERDAM, NL, vol. 1362, no. 1, 28 November 1997 (1997-11-28), pages 39-46, XP004276696 ISSN: 0925-4439
- ANDO YUKIO: "New therapeutic approaches for familial amyloidotic polyneuropathy (FAP)." AMYLOID : THE INTERNATIONAL JOURNAL OF EXPERIMENTAL AND CLINICAL INVESTIGATION : THE OFFICIAL JOURNAL OF THE INTERNATIONAL SOCIETY OF AMYLOIDOSIS. AUG 2003, vol. 10 Suppl 1, August 2003 (2003-08), pages 55-66, XP008059597 ISSN: 1350-6129
- ITOH N ET AL: "TRANSTHYRETIN PREALBUMIN IN THE PANCREAS AND SERA OF NEWLY DIAGNOSED TYPE I INSULIN-DEPENDENT DIABETIC PATIENTS" JOURNAL OF CLINICAL ENDOCRINOLOGY AND METABOLISM, vol. 74, no. 6, 1992, pages 1372-1377, XP008059577 ISSN: 0021-972X
- KATO M ET AL: "PLASMA AND CELLULAR RETINOID-BINDING PROTEINS AND TRANSTHYRETIN PREALBUMIN ARE ALL LOCALIZED IN THE ISLETS OF LANGERHANS IN THE RAT" PROCEEDINGS OF THE NATIONAL ACADEMY OF SCIENCES OF THE UNITED STATES OF AMERICA, vol. 82, no. 8, 1985, pages 2488-2492, XP002366590 ISSN: 0027-8424
- DATABASE WPI Section Ch, Week 200382 Derwent Publications Ltd., London, GB; Class B05, AN 2003-903939 XP002366896 & WO 03/094934 A1 (NIPRO CORP) 20 November 2003 (2003-11-20)
- PURKEY H E ET AL: "Evaluating the binding selectivity of transthyretin amyloid fibril inhibitors in blood plasma" PROCEEDINGS OF THE NATIONAL ACADEMY OF SCIENCES OF USA, NATIONAL ACADEMY OF SCIENCE, WASHINGTON, DC, US, vol. 98, no. 10, 8 May 2001 (2001-05-08), pages 5566-5571, XP002219441 ISSN: 0027-8424
- ALMEIDA MARIA ROSARIO ET AL: "Selective binding to transthyretin and tetramer stabilization in serum from patients with familial amyloidotic polyneuropathy by an iodinated diflunisal derivative" BIOCHEMICAL JOURNAL, vol. 381, no. Part 2, 15 July 2004 (2004-07-15), pages 351-356, XP002366589 ISSN: 0264-6021
- REFAI ESSAM ET AL: "Transthyretin constitutes a functional component in pancreatic beta-cell stimulus-secretion coupling" PROCEEDINGS OF THE NATIONAL ACADEMY OF SCIENCES OF THE UNITED STATES OF AMERICA, vol. 102, no. 47, November 2005 (2005-11), pages 17020-17025, XP002366591 ISSN: 0027-8424

## Description

### Field of the Invention

The present invention relates to methods and compositions for treating diabetes mellitus and methods for identifying compounds for treating diabetes mellitus.

### Background of the Invention

Transthyretin (TTR) is a tetrameric protein of identical 14 kDa subunits that is synthesized primarily in the liver and in the choroid plexus of the brain (*1*). It is a transport protein for thyroxin and, in association with the retinol-binding protein, for retinol. TTR has a complex equilibrium between different quaternary structures in serum (*2*), but exists mainly as a tetramer at a concentration of 160-380 mg/l, with only a small amount of TTR monomer *in vivo* in normal individuals (*3, 4*).

In type 1 diabetes (T1D) there is a specific destruction of the insulin secreting pancreatic β-cell. Although the exact molecular mechanisms underlying β-cell destruction are not known, sera from newly diagnosed T1D patients have been shown to increase the activity of voltage-gated L-type Ca²⁺-channels in β-cells (8). This results in unphysiological increases in [Ca²⁺]ᵢ upon depolarization and thereby β-cell apoptosis, effects that can be prevented by Ca²⁺-channel blockers. Ca²⁺-induced apoptosis may thus be involved in the complex autoimmune reaction associated with T1D. The key question has been what factor in T1D serum is responsible for the unphysiological changes in [Ca²⁺];. We have recently identified one such factor, apolipoprotein CIII (apoCIII) (*9*). ApoCIII is increased in serum from T1D patients and induces both increased [Ca²⁺]ᵢ and β-cell death. The effects of T1D sera and apoCIII on [Ca²⁺]ᵢ and β-cell apoptosis are abolished in the presence of an antibody against apoCIII. When considering the development of TID, it is of interest to note that the levels of proinsulin can be elevated in siblings of TID patients independent of HLA identity (*10*). This may reflect a compromised β-cell function prior to onset of TID. It is also of interest that the serum concentration of TTR tetramer is decreased in children with TID (*11*-*13*).

It is herein demonstrated that the TTR tetramer not only constitutes a functional component in normal pancreatic β-cell stimulus-secretion coupling but also preserves β-cell integrity. We also demonstrate that the concentration of total TTR is decreased in T1D, while that of the monomeric form is increased. An important observation was that the monomer neither constitutes a functional component in normal pancreatic β-cell stimulus-secretion coupling nor preserves β-cell integrity. Hence, it is likely that conversion of the tetrameric form of TTR to the monomeric form may serve as an important early step associated with the development of β-cell failure/destruction in T1D.

### Summary of the invention

In one aspect, the present invention provides TTR Tetramers for use in treating or preventing type I diabetes in a type I diabetic patient or in a patient at risk of developing type I diabetes.

In a further aspect, the present invention provides methods for identifying compounds for the treatment or prevention of type I diabetes comprising contacting pancreatic β cells with one or more test compounds and identifying those compounds that limit the reduction of TTR tetramers in the pancreatic β cells compared to control, wherein such compounds are candidate compounds for treating or preventing type I diabetes. In a further embodiment, the method further comprises preparing the compounds identified as preventing or limiting the reduction of TTR tetramers.

In a further aspect, the present invention provides methods for predicting risk of pancreatic β cell failure in a patient, comprising determining one or more of the following in pancreatic beta cells:
(a) an amount of monomeric transthyretin relative to control pancreatic β cells; and/or
(b) an amount of tetrameric transthyretin relative to control pancreatic β cells;
wherein the measurements made in (a) and (b) are used to determine a risk of pancreatic β cell failure.

In a further aspect, the present invention provides methods for predicting risk of type I diabetes in a patient, comprising determining one or more of the following in pancreatic beta cells derived from the patient:
(a) an amount of monomeric transthyretin relative to control pancreatic β cells; and/or
(b) an amount of tetrameric transthyretin relative to control pancreatic β cells;
wherein the measurements made in (a) and (b) are used to determine a risk of type I diabetes in the patient.

### Brief Description of the Figures

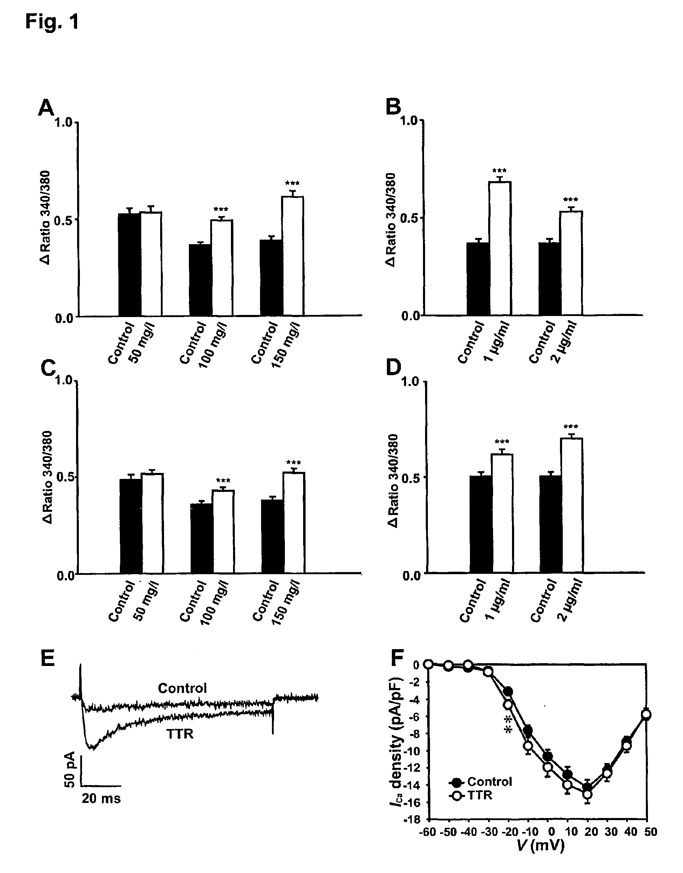

**Figure 1****. Effects on [Ca²⁺]ᵢ and voltage-gated Ca²⁺ currents A, C,** Cells incubated with total TTR. **B, D,** Cells incubated with the monomer. In **A** and **B**, the increase in [Ca²⁺]ᵢ is shown when cells were stimulated with glucose (**A**, *n*=48, 98, and 64, respectively for control cells and 46, 113, and 82 for TTR treated cells. **B**, *n*=50, 50 and 56, respectively for control cells and 73, 47, 74, respectively for TTR monomer treated cells). In **C** and **D**, the delta increase in [Ca²⁺]ᵢ is shown when cells were depolarised with KCl (C, *n*=48, 97, and 71, respectively for control cells and 44, 111 and 90 for TTR treated cells. **D**, *n*=83, 83 and 65, respectively for control cells and 83, 60 and 83 for TTR monomer treated cells). **E**, Sample whole-cell Ca²⁺ current traces, generated by a depolarizing voltage pulse (100 ms) to -20 mV from a holding potential of -70 mV, from a control cell (upper trace) and a cell incubated with TTR (lower trace). **F**, Whole-cell Ca²⁺ current-voltage relationships in pancreatic β-cells in the presence and absence of TTR. The TTR-treated β-cells (*n*=45) display larger Ca²⁺ currents than control cells (*n*=50) when they were depolarized to -20 mV.(**p<0.01, ***p<0.001).

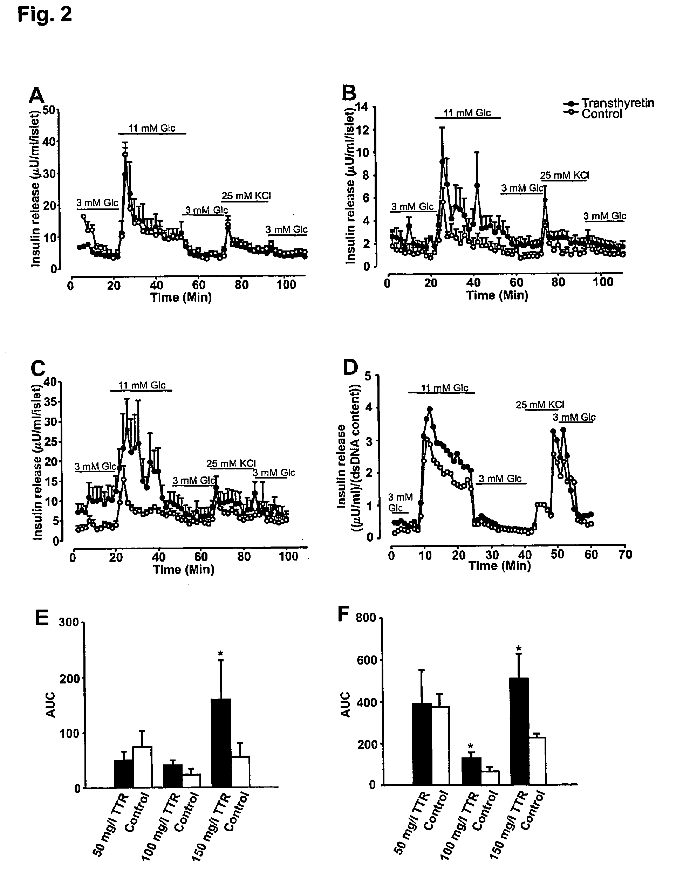

**Figure 2****. Effects of total TTR on insulin secretion.** Insulin release was measured in β-cells pre-incubated with **A**, TTR at 50 mg/l *(n*=5); **B**, at 100 mg/l (*n*=3); **C**, at 150 mg/l (*n*=5). **D** A representative trace (*n*=3) for insulin release measured in human islets exposed to 150 mg/l TTR. The insulin release from **A-C** expressed as AUC (area under the curve) for **E**, basal and **F**, glucose stimulated secretion. (*p<0.05, **p<0.01)

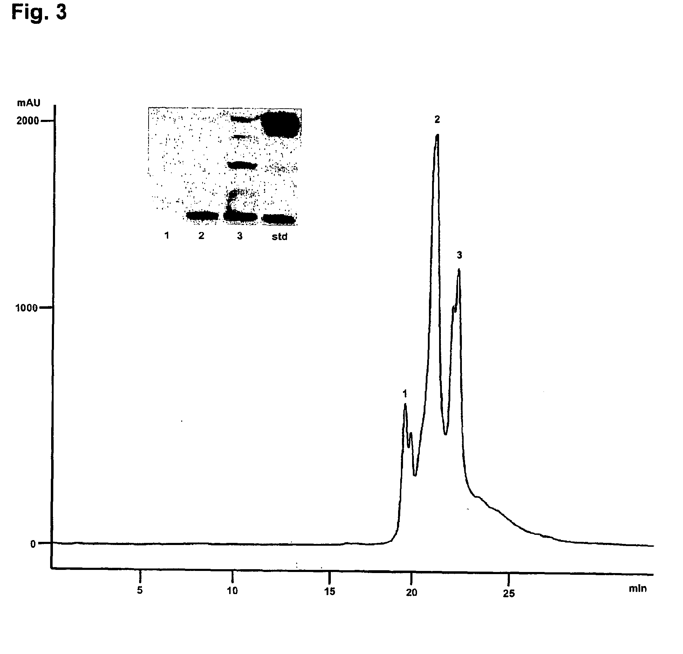

**Figure 3****. SDS-PAGE pattern of TTR.** Commercial TTR run on HPLC with a Vydac C18 column, acetonitrile (ACN) in 0.1% TFA; gradient 20-60% in 30 min, fractions 1, 2 and 3 were collected and run on SDS-PAGE, STD refers to the commercial TTR before HPLC run.

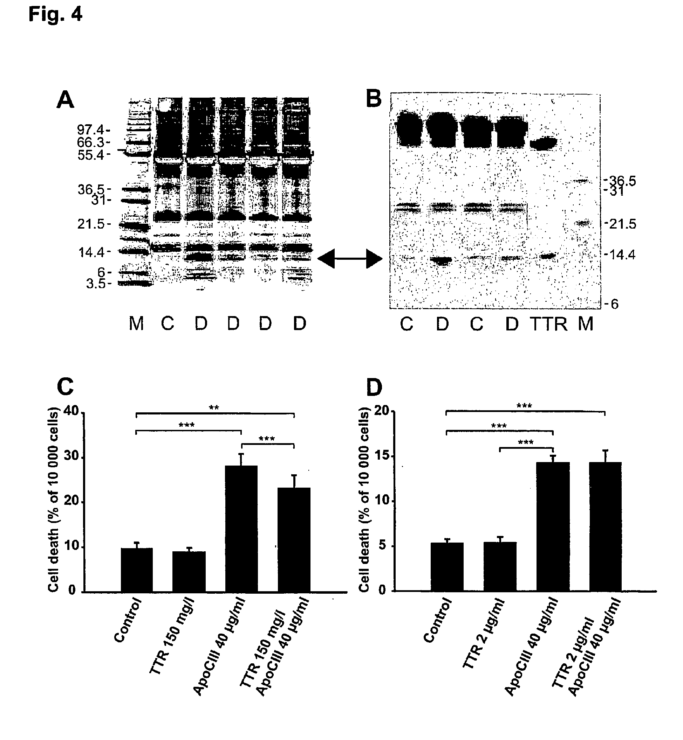

**Figure 4****. SDS-PAGE of sera from adults and children with T1D and healthy controls and effect of total TTR, TTR monomer ± apoCIII on cell death. A.** Five T1D sera (D) from adults and one control serum (C). **B.** Sera from two TID (D) and two control sera (C). The band for the monomeric form of TTR is marked with an arrow. In both **A** and **B**, lanes with commercial TTR and a broad range marker (M) were run. FACS analysis of pancreatic β-cells exposed to C. total TTR or Δ TTR monomer. TTR +/- (apoCIII or apoCIII alone (n=5). Cell death is expressed as the percentage of 10,000 counted cells.

### Detailed Description of the Invention

Within this application, unless otherwise stated, the techniques utilized may be found in any of several well-known references such as: Molecular Cloning: A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), "Guide to Protein Purification" in Methods in Enzymology (M.P. Deutshcer, ed., (1990) Academic Press, Inc.); A Manual of Basic Technique, 2nd Ed. (R.I. Freshney. 1987. Liss, Inc. New York, NY), Gene Transfer and Expression Protocols, pp. 109-128, ed. E.J. Murray, The Humana Press Inc., Clifton, N.J.), and the Ambion 1998 Catalog (Ambion, Austin, TX).

In one aspect, the present invention provides methods for treating or preventing type I diabetes, comprising administering an amount effective to treat or prevent type I diabetes of transthyretin ("TTR") tetramer to a type I diabetic patient or to a patient at risk of developing type I diabetes.

As disclosed herein, the TTR tetramer not only constitutes a functional component in normal pancreatic β-cell stimulus-secretion coupling but also preserves β-cell integrity. It is also disclosed that the concentration of total TTR is decreased in type I diabetes (TID), while that of the monomeric form is increased. An important observation was that the monomer neither constitutes a functional component in normal pancreatic β-cell stimulus-secretion coupling nor preserves β-cell integrity. Hence, it is likely that conversion of the tetrameric form of TTR to the monomeric form may serve as an important early step associated with the development of β-cell failure/destruction in TID.

As used herein, "transthyretin" refers to any known mammalian transthyretin protein, including but not limited to a protein comprising the amino acid sequence deposited as NCBI accession number **NP000362** (Human), NCBI accession number **NP036813** (Rat), or NCBI accession number **NP038725** (Mouse). As used herein, a "TTR tetramer" comprises 4 identical subunits of the TTR sequences disclosed above.

Human TTR tetramer is preferred for all therapeutic uses disclosed herein. For use in the therapeutic methods of the invention, it is not required that the TTR tetramer is purified away from TTR monomers or other aggregates, so long as the TTR tetramer is the predominant TTR form (ie: more than 50%). In preferred embodiments for therapeutic use, TTR tetramer comprises 55%, 60%, 65%, 70%, 75%, 80%, 85%, 90%, 95%, 98%, 99%, or 100% of the TTR used.

As used herein, an "amount effective" of the TTR tetramer is an amount that is sufficient to treat or prevent type I diabetes. An effective amount of the TTR tetramer that can be employed ranges generally between about 0.01 µg/kg body weight and about 10 mg/kg body weight, preferably ranging between about 0.05 µg/kg and about 5 mg/kg body weight. However dosage levels are based on a variety of factors, including the age, weight, sex, medical condition of the individual, the severity of the condition, the route of administration, and the particular compound employed. Thus, the dosage regimen may vary widely, but can be determined routinely by a physician using standard methods.

As used herein, "treating or preventing type I diabetes" includes any promotion of pancreatic β cell health, via any mechanism whereby TTR tetramers (a) promote a glucose-induced increase in cytoplasmic free Ca²⁺ concentration ([Ca²⁺]ᵢ) in the diabetic patient (treating) or patient at risk of developing type 1 diabetes (preventing); (b) promote a glucose-induced increase in insulin release from pancreatic β cells in the diabetic patient (treating) or patient at risk of developing type 1 diabetes (preventing); and/or (c) prevent or limit pancreatic β cell apoptosis. As used herein "preventing" means to slow or eliminate progression to type I diabetes in a patient at risk of developing type I diabetes. While not being bound by any specific mechanism, the inventors believe that TTR tetramer functions via a direct effect on the pancreatic β-cell voltage-gated L-type Ca²⁺ channel, and also by protecting against β-cell apoptosis, thus limiting the development of β-cell failure and destruction in TID patients or those at risk of developing TID, as well as optimizing appropriate insulin secretion from remaining pancreatic β cells in patients with type I diabetes. Patients at risk of developing type I diabetes include, but are not limited to, those with a genetic predisposition to type I diabetes.

The TTR polypeptides for use in the methods of the invention can further be derivatized to provide enhanced half-life, for example, by linking to polyethylene glycol or other such compounds, so long as such derivitization does not interfere with formation of the TTR tetramer. The polypeptides of the invention may comprise L-amino acids, D-amino acids (which are resistant to L-amino acid-specific proteases in vivo), a combination of D- and L-amino acids, and various "designer" amino acids (e.g., β-methyl amino acids, Cα-methyl amino acids, and Nα-methyl amino acids, etc.) to convey special properties. Synthetic amino acids include ornithine for lysine, and norleucine for leucine or isoleucine.

In addition, the polypeptides can have peptidomimetic bonds, such as ester bonds, to prepare polypeptides with novel properties. For example, a peptide may be generated that incorporates a reduced peptide bond, i.e., R₁-CH₂-NH-R₂, where R₁ and R₂ are amino acid residues or sequences. A reduced peptide bond may be introduced as a dipeptide subunit. Such polypeptides are resistant to protease activity, and possess an extended half-live in vivo.

The TTR polypeptides may be chemically synthesized or recombinantly expressed. Recombinant expression can be accomplished using standard methods in the art, generally involving the cloning of nucleic acid sequences capable of directing the expression of the polypeptides into an expression vector, which can be used to transfect or transduce a host cell in order to provide the cellular machinery to carry out expression of the polypeptides and formation of the tetramer. Such expression vectors can comprise bacterial or viral expression vectors, and such host cells can be prokaryotic or eukaryotic, preferably eukaryotic host cells in which tetramer formation can occur.

In one alternative, the polypeptides for use in the methods of the present invention are chemically synthesized. Synthetic polypeptides, prepared using the well-known techniques of solid phase, liquid phase, or peptide condensation techniques, or any combination thereof, can include natural and unnatural amino acids. Amino acids used for peptide synthesis may be standard Boc (Nα-amino protected Nα-t-butyloxycarbonyl) amino acid resin with the standard deprotecting, neutralization, coupling and wash protocols of standard solid phase procedure, or base-labile Nα-amino protected 9-fluorenylmethoxycarbonyl (Fmoc) amino acids. Both Fmoc and Boc Nα-amino protected amino acids can be obtained from Sigma, Cambridge Research Biochemical, or other chemical companies familiar to those skilled in the art. In addition, the polypeptides can be synthesized with other Nα-protecting groups that are familiar to those skilled in this art.

In a further alternative, TTR can be purified as a combination of tetramer and monomer from natural sources (such as human plasma), followed by isolation of the TTR tetramer away from the monomer. In another alternative, TTR is commercially available as a combination of monomeric and tetrameric forms (for example, from Sigma Chemical Co., St. Louis, MO, USA). Tetrameric TTR can be purified from these commercially available sources, using standard techniques, including but not limited to reverse-phase HPLC as described below (described in isolating monomeric TTR).

The TTR tetramer may be subjected to conventional pharmaceutical operations such as sterilization and/or may contain conventional adjuvants, such as preservatives, stabilizers, wetting agents, emulsifiers, buffers etc.

For administration, the TTR tetramer is ordinarily combined with one or more adjuvants appropriate for the indicated route of administration. The TTR tetramer may be admixed with lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, dextran sulfate, heparin-containing gels, and/or polyvinyl alcohol, and tableted or encapsulated for conventional administration. Alternatively, the TTR tetramer may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

The TTR tetramer may be administered by any suitable route, including orally, parentally, by inhalation spray, rectally, or topically in dosage unit formulations containing conventional pharmaceutically acceptable carriers, adjuvants, and vehicles. The term parenteral as used herein includes, subcutaneous, intravenous, intra-arterial, intramuscular, intrasternal, intratendinous, intraspinal, intracranial, intrathoracic, infusion techniques or intraperitoneally. Preferred embodiments for administration vary with respect to the condition being treated.

Isolated TTR tetramers can be prepared and purified as discussed herein, wherein the TTR tetramer is substantially free of any TTR monomer or other TTR tetramer binding protein. As used herein, the term "substantially free" means that the isolated TTR tetramers contain less that 2%, and more preferably less than 1.5%, 1%, 0.5%, 0.1%, 0.05%, 0.01%, 0.005%, 0.001%, 0.0005%, or 0.0001% TTR monomer or other TTR tetramer binding protein as determined by standard methods in the art, including but not limited to SDS-PAGE and reverse-phase HPLC. The TTR tetramer which does not contain detectable amounts of the monomer, can be prepared and purified as discussed herein.

Isolated TTR tetramers substantially free of agents used to separate protein components, such as polyacrylamide, agarose, and polymers/resins used to chromatographically separate protein components, can be prepared and purified as discussed herein. Methods for making the TTR tetramer are as described above.

The isolated TTR tetramer may be provided in a pharmaceutically acceptable carrier, such as those disclosed above. Such pharmaceutical compositions are preferred for therapeutic uses of the tetramer.

In a further aspect, the present invention provides methods for identifying compounds for the treatment or prevention of type I diabetes comprising contacting pancreatic β cells with one or more test compounds and identifying those compounds that limit the reduction of TTR tetramers in the pancreatic β cells compared to control, wherein such compounds are candidate compounds for treating or preventing type I diabetes.

As used herein, "pancreatic β cells" are any population of cells that contain pancreatic β islet cells. The cells can be obtained from any mammalian species, or may be present within the mammalian species when the assays are conducted in vivo. Such pancreatic β islet cell populations include the pancreas, isolated pancreatic islets of Langerhans ("pancreatic islets"), isolated pancreatic β islet cells, and insulin secreting cell lines. Methods for pancreatic isolation are well known in the art, and methods for isolating pancreatic islets, can be found, for example, in Cejvan et al., Diabetes 52:1176-1181 (2003); Zambre et al., Biochem. Pharmacol. 57:1159-1164 (1999), and Fagan et al., Surgery 124:254-259 (1998), and references cited therein. Insulin secreting cell lines are available from the American Tissue Culture Collection ("ATCC") (Rockville, MD). In a further embodiment where pancreatic β cells are used, they are obtained from ob/ob mice, which contain more than 95% β cells in their islets, and are commercially available.

In a preferred embodiment, the pancreatic β cells are derived from a patient with TID, or from an animal model of TID, and the amount of TTR tetramer, monomer, and/or ratio thereof are compared in pancreatic β cells contacted with the test compounds and control pancreatic β cells not contacted with the one or more test compounds. The contacting of the pancreatic β cells with the one or more test compounds can be in vitro, in vivo, or ex vivo. Test compounds that can be used include, but are not limited to polypeptides, antibodies, nucleic acids, or organic compounds, all of which can be made using standard methods in the art.

As used herein, "limit the reduction of TTR tetramers" refers to any mechanism whereby such reduction is limited, including but not limited to promoting tetramer formation and stabilizing TTR tetramers to reduce their conversion to monomeric TTR. In a preferred embodiment, the limit in the reduction of TTR tetramers compared to control is statistically significant. Any limit in the reduction of TTR tetramers compared to control can provide a benefit. In various embodiments, the amount of tetramer in test compound treated cells compared to diabetic control is at least 5%, 10%, 15%, 20%, 25%, 30%, 35%, 40%, 45%, 50%, 55%, 60%, 65%, 70%, 75%, 80%, 90%, 95%, or 100% higher.

Methods for determining the amount of TTR tetramer and/or monomer, or their ratio, can be any of those used in the art for making such measurements, including but not limited to SDS-PAGE and RP-HPLC, as disclosed below.

The method can further comprises preparing the compounds identified as limiting the reduction of TTR tetramers, using standard methods in the art.

The methods may further comprise administering to a type I diabetic patient or to a patient at risk of developing type I diabetes one or more compounds to limit the reduction of TTR tetramers. Such "limiting" includes any mechanism whereby such reduction is limited, including but not limited to promoting tetramer formation and stabilizing TTR tetramers to reduce their conversion to monomeric TTR. Such inhibitors can be identified using the methods described above.

In a further aspect, the present invention provides methods for predicting risk of pancreatic β cell failure in a patient, comprising determining one or more of the following in pancreatic beta cells:
(a) an amount of monomeric transthyretin relative to control pancreatic β cells; and/or
(b) an amount of tetrameric transthyretin relative to control pancreatic β cells;
wherein the measurements made in (a) and (b) are used to determine a risk of pancreatic β cell failure.

As disclosed below, the concentration of tetrameric TTR is decreased in TID, while that of the monomeric form is increased. The data below also indicate that conversion of the tetrameric form of TTR to the monomeric form may serve as an important early step associated with the development of β-cell failure/destruction in TID. Thus, methods to compare the amount of tetrameric and monomeric TTR to pancreatic beta cell controls (ie: either know TID controls or known normal pancreatic beta cells) can be used to predict a risk of pancreatic beta cell failure, which can be used, for example, to predict a risk of a patient developing TID. Thus, where the control is pancreatic β cells from a known T1D patient, an amount of monomeric TTR and/or tetrameric TTR similar to those from the T1D control indicate a risk of pancreatic β cell failure in the patient, while an amount of monomeric TTR less than and/or an amount of tetrameric greater than those from the T1D control indicate a lowered risk of pancreatic β cell failure. In a preferred embodiment, the differences or similarities between the test and control cells with respect to TTR levels is statistically significant. In one example, a prognosis of pancreatic β cell failure risk would be made where the amount of tetramer in the pancreatic beta cell compared to normal control is at least 5%,10%, 15%, 20%, 25%, 30%, 35%, 40%, 45%, 50%, 55%, 60%, 65%, 70%, 75%, 80%, 90%, 95%, or 100% lower.

In a further aspect, the present invention provides methods for predicting risk of type I diabetes in a patient, comprising determining one or more of the following in pancreatic beta cells derived from the patient:
(a) an amount of monomeric transthyretin relative to control pancreatic β cells; and/or
(b) an amount of tetrameric transthyretin relative to control pancreatic β cells;
wherein the measurements made in (a) and (b) are used to determine a risk of type I diabetes in the patient.

As disclosed below, the concentration of tetrameric TTR is decreased in T1D, while that of the monomeric form is increased. The data below also indicate that conversion of the tetrameric form of TTR to the monomeric form may serve as an important early step associated with the development of β-cell failure/destruction in T1D. Thus, methods to compare the amount of tetrameric and monomeric TTR to pancreatic beta cell controls (ie: either known T1D controls or known normal pancreatic beta cells) can be used to predict a risk of a patient developing T1D. Thus, where the control is pancreatic β cells from a known T1D patient, an amount of monomeric TTR and/or tetrameric TTR similar to those from the T1D control indicate a risk of the patient developing T1D, while an amount of monomeric TTR less than and/or an amount of tetrameric greater than those from the T1D control indicate a lowered risk of the patient developing T1D. In a preferred embodiment, the differences or similarities between the test and control cells with respect to TTR levels is statistically significant. In one example, a prognosis of T1D risk would be made where the amount of tetramer in the pancreatic beta cell compared to normal control is at least 5%, 10%, 15%, 20%, 25%, 30%, 35%, 40%, 45%, 50%, 55%, 60%, 65%, 70%, 75%, 80%, 90%, 95%, or 100% lower.

This prognostic method is especially useful for those patients at risk of developing T1D, such as those with a genetic predisposition to T1D.

In each of these prognostic aspects, the definition and possible sources of pancreatic β cells are as described above. Methods for detection and measurement of monomeric and tetrameric TTR are as described herein.

### Examples

We demonstrate below that the TTR tetramer not only constitutes a functional component in normal pancreatic β-cell stimulus-secretion coupling but also preserves β-cell integrity. We also demonstrate that the concentration of total TTR is decreased in type I diabetes (T1D), while that of the monomeric form is increased. An important observation was that the monomer neither constitutes a functional component in normal pancreatic β-cell stimulus-secretion coupling nor preserves β-cell integrity. Hence, it is likely that conversion of the tetrameric form of TTR to the monomeric form may serve as an important early step associated with the development of β-well failure/destruction in T1D**.**

### Methods

### Identification of TTR in sera from patients with T1D

Sera from T1D patients and control subjects were collected, identically sterile-processed, heat-inactivated by incubation at 56°C for 30 min and stored frozen at -20°C until used. Changes in [Ca²⁺]ᵢ were tested in β-cells when they were depolarized with 25 mM KCl. Those diabetic sera that induced a higher increase than sera from controls, were centrifuged and the supernatant was passed through a 0.45 mm sterile filter. Samples were loaded on Sep-Pak C18 pre-conditioned with 0.1% trifluoroacetic acid (TFA). After application of the sample, and a 5 ml wash with 0.1% TFA, proteins were eluted with 60% acetonitrile in 0.1% TFA. This procedure was repeated twice. The two fractions were pooled and the volume was reduced by lyophilization.

The lyophilized material was submitted to SDS-PAGE, with a reference of control sera from healthy subjects. The gel was silver stained and bands of the diabetic and non-diabetic sera were compared. The band at 14 kDa showed an up-regulation in diabetic sera.

Two SDS-PAGEs were run at the same time. One gel was stained with Coomassie Brilliant Blue and one was blotted on PVDF membrane and stained lightly. The band at 14 kDa was cut and in-gel digested with trypsin. After digestion, the material was run on a micro HPLC. Fractions were collected and mass fingerprinted by MALDI mass spectrometry, identifying TTR. This result was confirmed by amino acid sequencer analysis for 15 cycles.

### Media

The medium used for isolation of pancreatic β-cells as well as in the experiments was a HEPES buffer (pH 7.4) containing (in mM) 125 NaCl, 5.9 KCl, 1.2 MgCl₂, 1.28 or 2.56 CaCl₂ and 3.0 glucose. Bovine serum albumin was added at a concentration of 1mg/ml. RPMI 1640 culture medium, supplemented with 10% fetal bovine serum, was used for mouse cells and CMRL 1066 medium for human pancreatic islets.

### Preparation of cells

Pancreatic islets from ob/ob mice were isolated by a collagenase technique and mechanically disrupted into cells (*14*, *15*). Cells were either seeded onto plastic coverslips or used as suspensions. The human islets were isolated and quantified by the procedure of Ricordi et al (*16*).

### Measurements of insulin release

*Mouse*: Cells in suspension were incubated overnight with 50-, 100- or 150 mg/l TTR (Sigma) and 1 or 2 ug/ml monomer. Control cells were incubated with the vehicle (water) of TTR. Dynamics of insulin release were studied by perifusing islet cell aggregates mixed with Bio-Gel P4 polyacrylamide beads (Bio-Rad) in a 0.5 ml column at 37°C (17). The flow rate was 0.2 ml/min and 2 min fractions were collected and analyzed for insulin by radioimmunoassay using a rat insulin standard (Novo Nordisk).

*Human:* Islets were incubated overnight with 150 mg/l TTR, 2 ug/ml monomer or the vehicle. In these experiments a 0.27 ml column was used. The flow rate was 0.1 ml/min and 1 min fractions were collected and insulin was analyzed with ELISA (Mercodia). PicoGreen double-stranded DNA (dsDNA) quantification reagent kit (Molecular Probes) was used to quantify dsDNA in the human islets from each perifusion column. The insulin values were divided by the yielded factor and thus normalized for cell viability.

### Measurements of [Cₐ²⁺]ᵢ

Changes in [Ca²⁺]ᵢ were recorded in cells pre-incubated with 50-, 100 or 150 mg/l TTR and 1 or 2 ug/ml TTR monomer. Cells were attached to coverslips, loaded with 2 µM fura-2/AM (Molecular Probes) and mounted on an inverted epifluorescence microscope (Zeiss, Axiovert 135) connected to a Spex fluorolog-2 system for dual-wavelength excitations fluorimetri. The emissions of the two excitation wavelengths of 340 and 380 nm were used to calculate the fluorescence ratio 340/380 reflecting changes in [Ca²⁺]ᵢ. In order to compensate for possible variations in output of light intensity from the two monochromators, each experiment also included measurement of a 360/360 ratio. Every experiment was normalized by dividing all fluorescence ratios with the corresponding 360/360 ratio.

### Purification of the TTR monomer

TTR was run on a reverse phase (RP)-HPLC with an Everest 238 EV 54-C₁₈ (0.46 x 25 cm) column. The separation was made using a linear gradient of 20-60% acetonitrile in 0.1% TFA for 30 min at 1 ml/min. Fractions A1, A2 and A3 were collected and lyophilized. They were then run on a Novex Tricine gel 10-20%. Fraction A2 showed the mono form at a mass of 14 kDa (Fig.3).

### Patch clamp

Whole-cell Ca²⁺ currents were recorded in β-cells, incubated overnight with 150 mg/l TTR or the vehicle, by using the perforated-patch variant of whole-cell patch-clamp recording technique. Electrodes were filled with (in mM): 76 Cs₂SO₄, 1 MgCl₂, 10 KCl, 10 NaCl, and 5 Hepes (pH 7.35), as well as amphotericin B (0.24 mg/ml). The cells were bathed in a solution containing (in mM): 138 NaCl, 10 tetraethylammonium chloride, 10 CaCl₂, 5.6 KCl, 1.2 MgCl₂, 5 HEPES and 3 D-glucose (pH 7.4). Whole-cell currents induced by voltage pulses (from a holding potential of -70 mV to several clamping potentials from -60 to 50 mV in 10 mV increments, 100 ms, 0.5 Hz) were filtered at 1 kHz and recorded. All recordings were made with an Axopatch 200 amplifier (Axon Instruments, Foster City, California) at room temperature (about 22°C). Acquisition and analysis of data were done using the software program pCLAMP6 (Axon Instruments, Foster City, California).

### Flow cytometric analysis of cell death

Cells from ob/ob mice were cultured for 48 h in the presence of 2 µg/ml TTR monomer, 150 mg/l total TTR, 40 µg/ml apoCIII, TTR monomer or total TTR and apoCIII or the vehicle. The whole cell population was collected and stained with propidium iodide (PI) (BD PharMingen) and analysed on a FACscan using CELLQuest acquisition software (Becton Dickinson, Immunocytometry System). FACS gating, based on forward and side scatter, was used to exclude cellular debris and autofluorescence. Typically 10,000 cells were selected for analysis.

### Statistics

Statistical significance was evaluated by Student's t-test and p values <0.05 were considered significant. Data are presented as means ± SEM.

### Results

Transthyretin (TTR) is a transport protein mainly existing as a tetramer *in vivo*. TTR was found to promote glucose-induced increase in cytoplasmic free Ca²⁺ concentration ([Ca²⁺]ᵢ) and insulin release. This resulted from a direct effect on the pancreatic β-cell voltage-gated L-type Ca²⁺ channel. TTR also protected against β-cell apoptosis. The concentration of TTR tetramer was decreased, while that of a monomeric form was increased in type 1 diabetes (T1D). The monomer was without effect on glucose-induced insulin release and apoptosis. TTR tetramer thus constitutes a component in normal pancreatic β-cell function. Conversion of TTR tetramer to monomer may be involved in the development of β-cell failure/destruction in T1D.

Transthyretin (TTR) is a tetrameric protein of identical 14 kDa subunits that is synthesized primarily in the liver and in the choroid plexus of the brain (*1*). It is a transport protein for thyroxin and, in association with the retinol-binding protein, for retinol. TTR has a complex equilibrium between different quartenary structures in serum (2), but exists mainly as a tetramer at a concentration of 160-380 mg/l, with only a small amount of TTR monomer, *in vivo* in normal individuals (*3*, *4).* When the concentration of TTR is measured in serum samples by conventional methods, it is the tetrameric form that is detected. It is possible to estimate the amount of monomeric TTR both by SDS-PAGE, without reductive pre-boiling, and by reverse phase (RP)-HPLC. As shown in Fig 3, the monomer eluted before the tetramer on RP-HPLC (peak 2 and peak 3, respectively; peak 1 being non-TTR material). The monomeric form was verified by total mass determination, 14 kDa by MALDI mass spectrometry, and a monomer band on SDS-PAGE. A second HPLC fraction (peak 3) yielded a mass value of 50 kDa and on SDS-PAGE showed a monomer plus several aggregates, including tetramer. It appears possible that lack of complete dissociation of the tetramer upon SDS-PAGE may be due to the presence of some cross-linked or else chemically modified forms of TTR. Cys-10 of TTR has previously been shown to be sensitive to modification (2) and we therefore tested both the monomer and tetramer fractions from the HPLC by sequencer degradation, which however showed identical results for 15 cycles in both cases, suggesting that Cys-10 is not likely to be involved in the differential stability of the tetramer now observed.

We have measured changes in cytoplasmic free Ca²⁺ concentration, [Ca²⁺]i, in β-cells pre-treated with commercial TTR, which has both monomer and tetramer forms, and stimulated with glucose and KCl. TTR at a concentration of 100- and 150 mg/l induced a more pronounced increase in [Ca²⁺]ᵢ, subsequent to stimulation with glucose and KCl (Fig 1 A, C), compared to control, while 50 mg/l was without effect. Basal [Ca²⁺]ᵢ was not affected. At a concentration of 200 mg/l, TTR suppressed both glucose- and KCl-induced changes in [Ca²⁺]ᵢ (data not shown). In trying to elucidate the cellular mechanism underlying the increase in [Ca²⁺]ᵢ, the activity of voltage-gated Ca²⁺-channels was analysed in cells incubated with 150 mg/l TTR (Fig 1 E, F). Ca²⁺ currents registered from TTR-treated cells, depolarized to -20 mV, were significantly enhanced as compared to those from control cells (Fig 1 E, F). These data support that TTR acts on L-type Ca²⁺-channels as the effect occurred in the range of physiological depolarisations (5) and it is known that mouse β-cells contain exclusively L-type Ca²⁺-channels (6). The stimulus-secretion coupling in the pancreatic β-cell is a complex process where changes in [Ca²⁺]ᵢ serve as a key regulator of insulin release (7). To clarify the extent to which the TTR-induced changes in [Ca²⁺]ᵢ were paralleled by changes in insulin release, mouse β-cells were incubated overnight with commercial TTR and thereafter stimulated with glucose and KCl (Fig 2 A-C) in a perifusion system. There was no difference in insulin secretion, evaluated as area under the curve (AUC), in cells exposed to 50 mg/l TTR, whereas 100 mg/l gave an increase in glucose stimulated insulin release (p<0.05) and 150 mg/l in both basal (p<0.05) and glucose stimulated insulin release (p<0.05) (Fig 2 E, F). None of these TTR concentrations influenced KCl stimulated insulin release. We have also studied insulin release from human islets from three healthy subjects (donors to islet transplantation), incubated with 150 mg/l TTR, and the results were in accordance with those from mouse β-cells (Fig 2D). In accordance with the effects on [Ca²⁺]ᵢ, 200 mg/l TTR suppressed glucose and KCl-induced insulin release (data not shown). Although we have no explanation why 200 mg/l TTR drastically impairs β-cell function, exposure for prolonged periods of time under in vitro conditions to high concentrations of a transport protein like TTR may lead to unspecific interference with cell function. At the moment it is not clear to what extent an estimated in vivo concentration can be directly translated and used in an in vitro system. Our data suggest that such a translation is indeed not that straightforward.

In type 1 diabetes (T1D) there is a specific destruction of the insulin secreting pancreatic β-cell. Although the exact molecular mechanisms underlying β-cell destruction are not known, sera from newly diagnosed T1D patients have been shown to increase the activity of voltage-gated L-type Ca²⁺-channels in β-cells (8). This results in unphysiological increases in [Ca²⁺]ᵢ upon depolarization and thereby β-cell apoptosis, effects that can be prevented by Ca²⁺-channel blockers. Ca²⁺-induced apoptosis may thus be involved in the complex autoimmune reaction associated with TID. The key question has been what factor in T1D serum that is responsible for the unphysiological changes in [Ca²⁺]ᵢ. We have recently identified such a factor, namely apolipoprotein CIII (apoCIII) (*9*). ApoCIII is increased in serum from T1D patients and induces both increased [Ca²⁺]ᵢ and β-cell death. The effects of T1D sera and apoCIII on [Ca²⁺]ᵢ and β-cell apoptosis are abolished in the presence of an antibody against apoCIII. When considering the development of TID, it is of interest to note that the levels of proinsulin can be elevated in siblings of TID patients independent of HLA identity (*10*). This may reflect a compromised β-cell function prior to onset of TID.

It is of interest to note that the serum concentration of TTR tetramer is decreased in children with TID (*11*-*13*). Due to limited amount of serum material we could only measure the concentration of TTR, with kinetic nephelometry, in nine positive, three negative and ten control sera (Table 1). The levels were 116±11 mg/l in diabetic sera and 220±18 mg/l in control sera (p<0.001). Of the three negative diabetic sera (No 10-12, Table 1), one had a TTR level comparable with control sera (196 mg/l), while the other two were in the same low range as the positive diabetic sera. Knowing the possibility to estimate the amount of monomer, we analysed sera from both T1D and controls for SDS-PAGE patterns.

| Table 1 **Concentration of TTR in sera from T1D patients and healthy controls** | | |
|---|---|---|
| | TTR (diabetic sera) | TTR (control sera) |
| | (mg/l) | (mg/l) |
| 1 | 103 | 227 |
| 2 | 112 | 248 |
| 3 | 90 | 169 |
| 4 | 76 | 203 |
| 5 | 134 | 205 |
| 6 | 151 | 172 |
| 7 | 70 | 178 |
| 8 | 130 | 318 |
| 9 | 151 | 317 |
| 10 | 196 | 165 |
| 11 | 78 | |
| 12 | 105 | |
| Mean±SEM | 116±11 | 220±18 |

In sera from both adults (Fig 4A) and children (Fig 4B) with TID, the band representing the TTR monomer has a higher intensity on silver stained SDS-PAGE compared to sera from healthy controls. We managed to estimate the intensity of the band for the monomeric form in sera from ten of the twelve tested children and there was an up-regulation in six of them.

Our observation that the band on SDS-PAGE, representing TTR monomer, was of a higher intensity in TID patients, while the serum level of the tetramer was decreased, made us test the monomer on β-cell [Ca²⁺]ᵢ. The TTR monomer was purified from commercially available TTR by HPLC, and [Ca²⁺]ᵢ measurements were made on β-cells incubated with 1 and 2 µg/ml of the monomer. The concentrations were chosen based on the only study, to our knowledge, where the levels of monomer have been measured in serum from patients with familial amyloid polyneuropathy (FAP) and normal subjects (*4*). In FAP patients the concentration was 0.3±0.1 µg/ml and in the controls 0.6±0.2 µg/ml. Since our diabetic patients had a stronger band than the controls, we decided to take one concentration in the upper normal range and in addition one higher concentration. In cells exposed to 1- and 2 µg/ml of the monomer, glucose and depolarization with high K⁺ gave a more pronounced increase in [Ca²⁺]ᵢ compared to control cells (Fig 1 B, D). No difference in basal [Ca²⁺]ᵢ was observed. Insulin secretion, stimulated by glucose and KCl, was not affected by the monomer in either of the chosen concentrations, although basal insulin secretion was significantly higher in cells treated with 2 µg/ml monomer. An increased basal insulin secretion may reflect impaired β-cell function. Interestingly, whereas the TTR tetramer (150 mg/l) protected against apoCIII induced β-cell apoptosis, the monomer (2 µg/ml) was without effect (Fig 4 C and D).

### References and Notes

1. D. R. Soprano, J. Herbert, K. J. Soprano, E. A. Schon, D. S. Goodman, J Biol Chem 260, 11793-8 (1985).
2. T. Pettersson, A. Carlstrom, H. Jornvall, Biochemistry 26, 4572-83 (1987).
3. C. C. Blake, M. J. Geisow, S. J. Oatley, B. Rerat, C. Rerat, J Mol Biol 121, 339-56 (1978).
4. Y. Sekijima et al., Amyloid 8, 257-62 (2001).
5. P. Rorsman, G. Trube, J Physiol 374, 531-50 (1986).
6. F. M. Ashcroft, P. Rorsman, Prog Biophys Mol Biol 54, 87-143 (1989).
7. P. O. Berggren et al., Adv Exp Med Biol 334, 25-45 (1993).
8. L. Juntti-Berggren et al., Science 261, 86-90 (1993).
9. L. Juntti-Berggren et al., Proc Natl Acad Sci U S A 101,10090-10094 (2004).
10. S. G. Hartling, F. Lindgren, G. Dahlqvist, B. Persson, C. Binder, Diabetes 38, 1271-4 (1989).
11. M. Gebre-Medhin, U. Ewald, T. Tuvemo, Acta Paediatr Scand 74, 961-5 (1985).
12. A. M. Kobbah, K. Hellsing, T. Tuvemo, Acta Paediatr Scand 77, 734-40 (1988).
13. S. K. Jain, R. McVie, J. Duett, J. J. Herbst, Horm Metab Res 25, 102-4 (1993).
14. A. Lermnark, Diabetologia 10, 431-8 (1974).
15. P. Arkhammar, P. O. Berggren, P. Rorsman, Biosci Rep 6, 355-61 (1986).
16. C. Ricordi, P. E. Lacy, E. H. Finke, B. J. Olack, D. W. Scharp, Diabetes 37, 413-20 (1988).
17. T. Kanatsuna, A. Lernmark, A. H. Rubenstein, D. F. Steiner, Diabetes 30, 231-4 (1981).

## Claims

1. Use of an effective amount of transthyretin tetramer for the manufacture of a medicament to treat type I diabetes or to prevent type I diabetes in a type I diabetic patient, or a patient at risk of developing type I diabetes.

2. The use of claim 1, wherein the transthyretin tetramer is substantially devoid of transthyretin monomers.

3. The use of claim 1 or 2, wherein the transthyretin tetramer comprises mammalian transthyretin.

4. The use of claim 3, wherein the mammalian transthyretin is human transthyretin.

5. The use of any of claims 1 to 4, wherein type I diabetes in a type I diabetic patient is treated.

6. The use of any of claims 1 to 4, wherein type I diabetes is prevented in a patient at risk of developing type I diabetes.

7. A method for identifying compounds for the treatment or prevention of type I diabetes, comprising contacting pancreatic β cells with one or more test compounds and identifying those test compounds that limit the reduction of TTR tetramers in the pancreatic β cells compared to control, wherein such test compounds are candidate compounds for treating or preventing type I diabetes.

8. The method of claim 7, further comprising synthesizing the candidate compounds.

9. The method of claim 7, wherein the pancreatic β cells are from a patient with type 1 diabetes or from an animal model of type 1 diabetes.

10. The method of claim 7, wherein the pancreatic β cells are from ob/ob mice.

11. A method for predicting risk of pancreatic β cell failure, comprising detecting one or more of the following in pancreatic beta cells, wherein said cells are obtained from any mammalian species:
(a) an amount of monomeric transthyretin relative to control pancreatic β cells; and/or
(b) an amount of tetrameric transthyretin relative to control pancreatic β cells;
wherein the measurements made in (a) and (b) are used to determine a risk of pancreatic β cell failure.

12. The method of claim 11, wherein the method is used to predict a risk of development of type I diabetes.

13. A method for predicting risk of type I diabetes in a patient, comprising detecting one or more of the following in pancreatic beta cells, wherein said cells are derived from said patient:
(a) an amount of monomeric transthyretin relative to control pancreatic β cells; and/or
(b) an amount of tetrameric transthyretin relative to control pancreatic β cells;
wherein the measurements made in (a) and (b) are used to determine a risk of type I diabetes in the patient.

14. Transthyretin tetramer for use in treating or preventing type I diabetes in a type I diabetic patient, or a patient at risk of developing type I diabetes.

15. The transthyretin tetramer for use according to claim 14 wherein the transthyretin tetramer comprises human transthyretin.

## Patentansprüche

1. Verwendung einer effektiven Menge Transthyretin Tetramer für die Herstellung eines Medikaments zum Behandeln von Typ 1 Diabetes oder zum Verhindern von Typ 1 Diabetes in einem Typ 1 diabetischen Patient, oder einem Patienten mit dem Risiko, Typ 1 Diabetes zu entwickeln.

2. Die Verwendung des Anspruchs 1, wobei das Transthyretin Tetramer im Wesentlichen frei von Transthyretin Monomeren ist.

3. Die Verwendung des Anspruchs 1 oder 2, wobei das Transthyretin Tetramer Säugetier-Transthyretin umfasst.

4. Die Verwendung des Anspruchs 3, wobei das Säugetier-Transthyretin humanes Transthyretin ist.

5. Die Verwendung eines der Ansprüche 1 bis 4, wobei Typ 1 Diabetes in einem Typ 1 diabetischen Patienten behandelt wird.

6. Die Verwendung eines der Ansprüche 1 bis 4, wobei Typ 1 Diabetes in einem Patienten mit dem Risiko, Typ 1 Diabetes zu entwickeln, verhindert wird.

7. Methode zum Identifizieren von Verbindungen für die Behandlung oder Verhinderung von Typ 1 Diabetes, umfassend das Inkontaktbringen von pankreatischen β-Zellen mit einer oder mehreren Testverbindungen und das Identifizieren solcher Testverbindungen, die die Reduktion von TTR Tetrameren in den pankreatischen β-Zellen verglichen mit einer Kontrolle limitiert, wobei solche Testverbindungen Kandidatverbindungen für das Behandeln oder Verhindern von Type 1 Diabetes sind.

8. Die Methode des Anspruchs 7, weiterhin umfassend das Synthetisieren von Kandidatverbindungen.

9. Die Methode des Anspruchs 7, wobei die pankreatischen β-Zellen von Patienten mit Typ 1 Diabetes oder von einem Tiermodell von Typ 1 Diabetes sind.

10. Die Methode des Anspruchs 7, wobei die pankreatischen β-Zellen von ob/ob Mäusen sind.

11. Methode für das Vohersagen des Risikos des Versagens von pankreatischen β-Zellen, umfassend das Detektieren einer oder mehrerer der folgenden pankreatischen beta Zellen, wobei diese Zellen von irgendeiner Säugetierspezies erhalten wird:
a) eine Menge von monomerischem Transthyretin relativ zu pankreatischen β-Kontrollzellen; und/oder
b) eine Menge von tetramerischem Transthyretin relativ zu pankreatischen β-Kontrollzellen;
wobei die Messungen ausgeführt in a) oder b) verwendet werden, um das Risiko des Versagens von pankreatischen β-Zellen zu bestimmen.

12. Die Methode des Anspruchs 11, wobei die Methode verwendet wird, um das Risiko der Typ 1 Diabetes Entwicklung vorherzusagen.

13. Methode für das Vorhersagen des Risikos von Typ 1 Diabetes in einem Patienten, umfassend das Detektieren einer oder mehrerer der folgenden pankreatischen beta Zellen, wobei diese Zellen von besagtem Patienten abstammen:
a) eine Menge von monomerischem Transthyretin relativ zu pankreatischen β-Kontrollzellen; und/oder
b) eine Menge von tetramerischem Transthyretin relativ zu pankreatischen β-Kontrollzellen;
wobei die Messungen ausgeführt in a) oder b) verwendet werden, um das Risiko von Typ 1 Diabetes in dem Patienten zu bestimmen.

14. Transthyretin Tetramer für die Verwendung im Behandeln oder Verhindern von Typ 1 Diabetes in einem Typ 1 diabetischen Patienten oder einem Patienten mit dem Risiko, Typ 1 Diabetes zu entwickeln.

15. Das Transthyretin Tetramer für die Verwendung gemäß dem Anspruch 14, wobei das Transthyretin Tetramer humanes Transthyretin umfasst.

## Revendications

1. Utilisation d'une quantité efficace de tétramère de transthyrétine pour la préparation d'un médicament pour traiter le diabète de type I ou pour prévenir le diabète de type I chez un patient diabétique de type I, ou un patient ayant un risque de développer un diabète de type I.

2. Utilisation selon la revendication 1, dans laquelle le tétramère de transthyrétine est substantiellement exempt de monomères de transthyrétine.

3. Utilisation selon la revendication 1 ou 2, dans laquelle le tétramère de transthyrétine comprend de la transthyrétine mammalienne.

4. Utilisation selon la revendication 3, dans laquelle la transthyrétine mammalienne est la transthyrétine humaine.

5. Utilisation selon l'une quelconque des revendications 1 à 4, dans laquelle on traite le diabète de type I chez un patient diabétique de type I.

6. Utilisation selon l'une quelconque des revendications 1 à 4, dans laquelle le diabète de type I est évité chez un patient ayant un risque de développer un diabète de type I.

7. Procédé pour l'identification de composés pour la traitement ou la prévention du diabète de type I, comprenant la mise en contact de cellules β pancréatiques avec un ou plusieurs composés d'essai et l'identification des composés d'essai qui limitent la réduction des tétramères de TTR dans les cellules β pancréatiques par comparaison avec un témoin, tandis que de tels composés d'essai sont des composés candidats pour le traitement ou la prévention du diabète de type I.

8. Procédé selon la revendication 7, comprenant en outre la synthèse des composés candidats.

9. Procédé selon la revendication 7, dans lequel les cellules β pancréatiques proviennent d'un patient ayant un diabète de type 1 ou d'un modèle animal de diabète de type I.

10. Procédé selon la revendication 7, dans lequel les cellules β pancréatiques proviennent de souris ob/ ob.

11. Procédé pour la prédiction du risque de défaillance de cellules β pancréatiques, comprenant la détection d'un ou plusieurs des éléments suivants dans des cellules bêta pancréatiques, tandis que lesdites cellules sont obtenues à partir une espèce mammalienne quelconque:
(a) une quantité de transthyrétine monomère par rapport à des cellules β pancréatiques témoin; et/ ou
(b) une quantité de transthyrétine tétramère par rapport à des cellules β pancréatiques témoin;
tandis que les mesures effectuées dans (a) et (b) sont utilisées pour déterminer un risque de défaillance des cellules β pancréatiques.

12. Procédé selon la revendication 11, dans lequel le procédé est utilisé pour prédire un risque de développement du diabète de type I.

13. Procédé pour la prédiction d'un risque de diabète de type I chez un patient, comprenant la détection d'un ou plusieurs des éléments suivants dans des cellules bêta pancréatiques, tandis que lesdites cellules sont dérivées dudit patient:
(a) une quantité de transthyrétine monomère par rapport à des cellules β pancréatiques témoin; et/ ou
(b) une quantité de transthyrétine tétramère par rapport à des cellules β pancréatiques témoin;
tandis que les mesures effectuées dans (a) et (b) sont utilisées pour déterminer un risque de diabète de type I chez le patient.

14. Tétramère de transthyrétine pour utilisation dans le traitement ou la prévention du diabète de type I chez un patient diabétique de type I, ou un patient ayant un risque de développer un diabète de type I.

15. Tétramère de transthyrétine pour utilisation selon la revendication 14, dans laquelle le tétramère de transthyrétine comprend de la transthyrétine humaine.
